(19) Europäisches Patentamt / European Patent Office / Office européen des brevets

(11) **EP 2 306 982 B1**

(12) **EUROPEAN PATENT SPECIFICATION**

(45) Date of publication and mention of the grant of the patent:
**07.01.2015 Bulletin 2015/02**

(51) Int Cl.:
***A61K 9/20*** *(2006.01)*          ***A61K 31/505*** *(2006.01)*
***A61P 3/06*** *(2006.01)*

(21) Application number: **08776549.1**

(86) International application number:
**PCT/IB2008/052595**

(22) Date of filing: **27.06.2008**

(87) International publication number:
**WO 2009/156796 (30.12.2009 Gazette 2009/53)**

(54) **PHARMACEUTICAL COMPOSITIONS OF ROSUVASTATIN CALCIUM**

PHARMAZEUTISCHE ZUSAMMENSETZUNGEN VON ROSUVASTATIN-CALCIUM

COMPOSITIONS PHARMACEUTIQUES DE ROSUVASTATINE CALCIUM

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**13.04.2011 Bulletin 2011/15**

(73) Proprietor: **Abdi Ibrahim Ilac Sanayi ve Ticaret Anonim Sirketi**
**Istanbul (TR)**

(72) Inventors:
- **FARSHI, Farhad Sayyad**
  **Bahcesehir 34555 Istanbul (TR)**
- **AVCI, Recep**
  **Bahcesehir 34555 Istanbul (TR)**
- **APARI, Serdar**
  **Bahcesehir 34555 Istanbul (TR)**

(56) References cited:
| | |
|---|---|
| EP-A- 1 905 424 | WO-A-03/092729 |
| WO-A-2004/056395 | WO-A-2005/046662 |
| WO-A-2006/134604 | WO-A-2008/005543 |
| WO-A-2008/062476 | |

- **M. KOBAYASHI ET AL.: "Preventive effects of bicarbonate on cerivastatin-induced apoptosis" INTERNATIONAL JOURNAL OF PHARMACEUTICS, vol. 341, 2007, pages 181-188, XP022166302**
- **ANONYMOUS: "Guidance for Industry: Waiver of In Vivo Bioavailability and Bioequivalence Studies for Immediate-Release Solid oral Dosage Forms Based on a Bopharmaceutics Classification System. August 2000 (FDA)", FDA CONSUMER, CE, FOOD AND DRUG ADMINISTRATION, ROCKVILLE, MD, 1 August 2000 (2000-08-01), pages 1-16, XP003031467, ISSN: 0362-1332**

**Description**

The present invention is related to pharmaceutical compositions of rosuvastatin calcium with alkali metal -carbonate -bicarbonate or a mixture therof which have effects on dissoltion profiles in 0.1 N HCl medium provided that using of sodium carbonate anhydrous should be present in the range of 0,5 % to 2 % by weight of the pharmaceutical composition and molar ratio is in the range of 1 : 0.43-1.75 (Rosuvastatin calcium : alkali metal-carbonate -bicarbonate or a mixture therof). Pharmaceutical composition is preferably oral dosage form. Oral dosage form is preferably tablet and could be other oral dosage forms such as capsule, pellet, minitablet etc.

**Background Art**

EP 521471 which is basic patent of rosuvastatin and discloses rosuvastatin as an inhibitor of HMG-CoA reductase.

In the patent of EP 0547000 it is described that formulations of HMG-CoA inhibitors with alkaline substances including sodium carbonate and/or calcium carbonate in the range of from 0,1 % to 60 % . However EP 0547000 does not denote that effects of the percentage or molar ratio of alkaline substances in rosuvastatin formulations on dissolution profiles in 0,1 N HCl environment. EP 0547000 points out that only effects on stability and intestinal absorbtion.

In the patent of EP 1223918 a protection is determined that using of a tribasic phosphate salt, in which the cation is multivalent, in pharmaceutical compositions of rosuvastatin. Actually, the rationale of using of tribasic phosphate salt in which multivalent cation dissolution profilesin 0,1 N HCl environment are different from other alkanizing agents. Therefore, for providing bioequivalence, if generics use alkanizing agent other than tribasic phosphate salt in which multivalent cation they could not reach desired dissolution profiles in 0,1 N HCl environment and could not achieve bioequivalance. According to information of DailyMed web site, in Crestor ® tablet formulations in 5-10-20 and 40 mg, tribasic calcium phosphate is used as a tribasic phosphate salt which is included group of cation is multivalent.

EP 1905424 discloses that the percentage of alkanizing agent including sodium carbonate may vary from about 1 to about 50 by weight of the composition. EP 1905424 does not disclose effect of alkanizing agents on dissolution profiles and does not point out any percentage or molar ratio to achieve critical dissolution in 0,1 N HCl environment. Sodium carbonate *per se* could be used to stabilize as it is explained in that patent, but it has important effects on dissolution profiles in 0,1 N HCl environment according to our invention.

In patent application of WO 2008/062476 sodium carbonate is used as inorganic salt of monovalent cation in rosuvastatin formulations. But there is no any disclosing as to effects of using of sodium carbonate on dissolution profiles in 0,1 N HCl environment Claim 7 discloses that using of inorganic salt of monovalent cation within the range of 0.01 % to 10 % by weight of the composition without denoting effects of sodium carbonate on dissolution profile.

**Detailed Description of Invention**

In generally, dissolution environment is held by the generics as medium of pH 6.6 citrate to accomplish of bioequivalence since FDA (Food and Drug Administration) hold that dissolution environment of rosuvastatin calcium is pH 6.6 in vitro (Dissolution Methods of Drug Products). Using of alkali or earth alkali metal - carbonate -bicarbonate or a mixture of therof in any percentage in pharmaceutical composition of rosuvastatin or in any molar ratio with rosuvastatin , dissolution profiles of reference tablets and test tablets are the same or identical in pH 6.6 environment. In other words, in pH 6.6 environment, alkali or earth alkali metal -carbonate - bicarbonate or a mixture of therof in any percentage in pharmaceutical composition of rosuvastatin or in any molar ratio with rosuvastatin has no any effect on dissolution profiles and dissolution profiles are not depended on amount of alkali or earth alkali metal-carbonate -bicarbonate . In rosuvastatin tablet formulations, only holding this result is not enough to carry out bioequivalence studies and to achieve bioequivalence since reference tablet is dissolved as from time of intaking to stomach. In other words, after administration oral solid dosage form is released upon contact with stomach environment before the absorbtion. Therefore it is important to consider the dissolving in 0,1 N HCl environment. The medium of stomach is 0,1 N HCl and dissolution profiles of generic product should overlap with dissolution profiles of reference product in 0,1 N HCl environment. Thus overlapping of dissolution profiles in pH 6.6 environment alone is not sufficient. According to this invention, most probably, test product shall be bioequivalence with reference product as trademark of Crestor ®.

In this application we unexpecdetly and surprisingly find out that a special molar ratio between rosuvastatin calcium and alkali metal -carbonate -bicarbonate or a mixture therof provides a dissolution profile with a similarity factor greater then 50 in 0.1 N HCl dissolution media compared with Crestor ®. Molar ratio is in the range of 1 : 0.43-1.75 (Rosuvastatin calcium : alkali metal -carbonate -bicarbonate or a mixture therof). Alkali metals are selected from Na or K. Preferably sodium carbonate anhydrous is used.

Another aspect of this disclosure is related to effects of alkali metal - carbonate -bicarbonate or a mixture therof , which are used in a specific range, on dissolution profiles of rosuvastatin calcium in medium of 0.1 N HCl. According to

this, in medium of 0.1 N HCl, dissolution profiles of rosuvastatin calcium tablets including alkali metal -carbonate -bicarbonate or a mixture therof , which are used in a specific range by the weight of pharmaceutical composition, are the same with reference tablets that are sold on the market with trademark of Crestor ® . To overlap of dissolution profiles of reference tablet and test tablet in medium of 0.1 N HCl ,alkali metal -carbonate -bicarbonate or a mixture of therof are used in the range of % 0,5 to % 2, preferably 0.6 % to 1,4 % by weight of the tablet.

In another aspect of the invention is pharmaceutical composition comprising at least one pharmaceutically suitable excipient. Excipient could be a filler or a disintegrating agent or a lubricant or another suitable excipient or mixture of thereof.

Yet another aspect is a pharmaceutical composition comprising rosuvastatin calcium including alkali metal -carbonate -bicarbonate or a mixture therof characterized in that alkali metal -carbonate -bicarbonate or a mixture of therof are used in the range of 0.5 % to 2 % by weight of the pharmaceutical composition and the active ingredient has an in vitro dissolution profile in 0,1 N HCl environment with a similarity factor (f2) of at least 50 to 100 compared to the reference dissolution profile.

The similarity factor f2 is a measurement of the similarity through a point by point comparison as shown in equation 1.

$$f_2 = 50 * \log \frac{100}{\sqrt{1 + \frac{1}{n} \sum_{t=1}^{n} (R_t - T_t)^2}}$$

(Equation 1)

n: is the number of sampling time points

$R_t$ : is the amount drug released from a *reference* batch at time t

$T_t$: is the amount drug released from a *test* batch at time t.

Generally, $f_2$ values greater than 50 ensure sameness of the performance of the reference product and test product.

Pharmaceutical composition is preferably oral dosage formulations such as tablet, capsule, pellet, minitablet etc. Especially preferred oral dosage formulation is tablet.

Using of alkali metal -carbonate -bicarbonate or a mixture therof within the range of 0,5 % to 2 % by weight of composition and within the molar ratio in the range of 1 : 0.43-1.75 (Rosuvastatin calcium : alkali metal -carbonate -bicarbonate or a mixture therof) also do not bring about impurities such as lactones.

It is unexpectedly and surprisingly found that using of alkali metal - carbonate -bicarbonate or a mixture therof in tablet formulations of rosuvastatin calcium within the range of 0,5 % to 2 % by weight of the tablet, preferably 0,6 % to 1,4 % and within the molar ratio in the range of 1 : 0.43-1.75 (Rosuvastatin calcium : alkali metal -carbonate -bicarbonate or a mixture therof), dissolution profiles in the 0,1 N HCl environment is about identical with reference tablet, known as Crestor ®. In rosuvastatin calcium formulations, effects of using of alkali metal - carbonate -bicarbonate or a mixture therof , which is in mentioned specific ranges, its effect on dissolution profiles in 0,1 N HCl environment is unexpected result, since it is not known by the prior art and it is only known that using of alkali or earth alkali metal -carbonate -bicarbonate or a mixture therof in rosuvastatin calcium formulations, it prevents occurring impurities such aslactone.

Any sutiable dissolution method could be used to reach dissolution profiles. In this invention USP method-1 (basket) is preferred and used. However USP method-1 (basket) is not exhaustive and other eligible methods such as but not limited USP method-II (paddle) could be contemplated.

Pharmaceutical composition is preferably prepared as a tablet that exhibits a dissolution profile as less than or equal to 85 % of the total amount of rosuvastatin calcium is released in 30 minutes after combining the tablet with 900 ml of a dissolution medium at 37°C ± 0.5°C through using of USP method 1 (basket) and basket speed is 100 rpm

Pharmaceutical composition has a dissolution profile as rosuvastatin calcium is released in the range of 15 % to 25 % in 5 minutes, 40 % to 50 % in 10 minutes, 60 % to 70 % in 15 minutes, 70 % to 80 % in 20 minutes, 80 % to 90 % in 30 minutes, 85 % to 95 % in 45 minutes and 90 to 100 % in 60 minutes after association of 900 ml of a dissolution medium at 37°C ± 0.5°C and USP method 1 (basket) and basket speed is 100 rpm.

Alkali metal carbonates are preferably $Na_2CO_3$or $K_2CO_3$. In this invention sodium carbonate anhydrous is preferred and used.

## Example 1

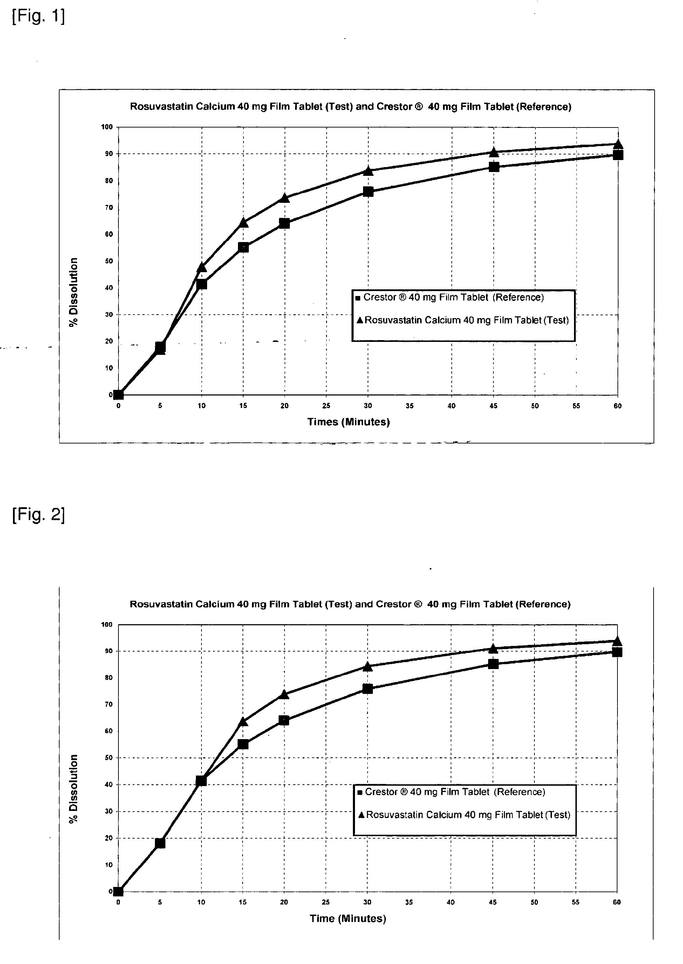

Rosuvastatin calcium test tablet includes sodium carbonate anhydrous as within the molar ratio in the range of 1 : 1.17 (Rosuvastatin calcium : sodium carbonate anhydrous) is released in 0,1 N HCl environment under conditions of 900 ml of a dissolution medium at 37°C ±0.5°C, USP method 1 (basket), 100 rpm basket speed wherein tablet exhibits a dissolution profile (Figure 1 and Table 1). Under mentioned conditions f2 value is 57,9 . At the same time sodium carbonate anhydrous is 1,34 % by weight of tablet.

[Table 1]
[Table ]

### Table 1 : Comparison of Test and Reference Tablets (molar ratio is 1:1.17 as rosuvastatin calcium : sodium carbonate anhydrous)

| Time (Minutes) | Dissolved % | |
|---|---|---|
| | Crestor ® 40 mg Film Tablet (Reference) | Rosuvastatin Calcium 40 mg Film Tablet (Test) |
| 5 | 18,1 | 17,0 |
| 10 | 41,6 | 47,9 |
| 15 | 55,1 | 64,5 |
| 20 | 64,1 | 73,7 |
| 30 | 75,9 | 83,8 |
| 45 | 85,2 | 90,8 |
| 60 | 89,8 | 93,9 |

### Example 2

Rosuvastatin calcium test tablet includes sodium carbonate anhydrous as within the molar ratio in the range of 1 : 1.75 (Rosuvastatin calcium : sodium carbonate anhydrous) is released in 0,1 N HCl environment under conditions of 900 ml of a dissolution medium at 37°C ±0.5°C, USP method 1 (basket), 100 rpm basket speed wherein tablet exhibits a dissolution profile (Figure 1 and Table 1). At the same time sodium carbonate anhydrous is 2 % by weight of tablet (Figure 2 and Table 2). Under mentioned conditions f2 value is 59,1 .

[Table 2]
[Table ]

### Table 2 : Comparison of Test and Reference Tablets (molar ratio is 1:1.75 as rosuvastatin calcium : sodium carbonate anhydrous)

| Time (Minutes) | Dissolved % | |
|---|---|---|
| | Crestor ® 40 mg Film Tablet (Reference) | Rosuvastatin Calcium 40 mg Film Tablet (Test) |
| 5 | 18,1 | 18 |
| 10 | 41,6 | 41,5 |
| 15 | 55,1 | 63,7 |
| 20 | 64,1 | 73,9 |
| 30 | 75,9 | 84,4 |
| 45 | 85,2 | 91,1 |
| 60 | 89,8 | 93,9 |

### Example 3

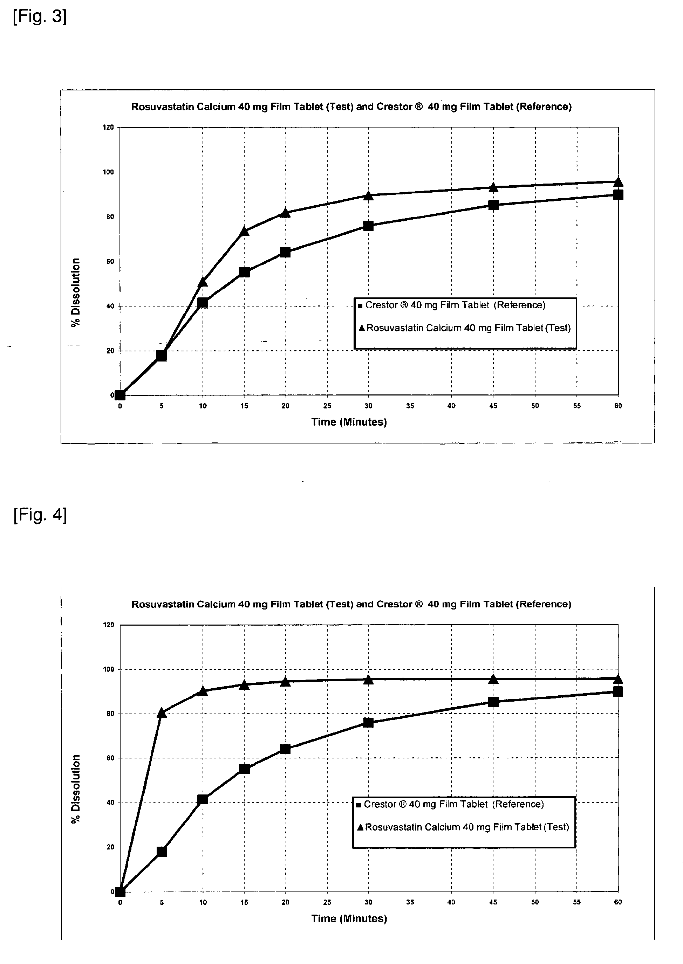

Rosuvastatin calcium test tablet includes sodium carbonate anhydrous as within the molar ratio in the range of 1 : 2.35 (Rosuvastatin calcium : sodium carbonate anhydrous) is released in 0,1 N HCl environment under conditions of 900 ml of a dissolution medium at 37°C ±0.5°C, USP method 1 (basket), 100 rpm basket speed wherein tablet exhibits a dissolution profile (Figure 1 and Table 1). At the same time sodium carbonate anhydrous is 2.65 % by weight of tablet (Figure 3 and Table 3). Under mentioned conditions f2 value is 45,7 .
[Table 3]
[Table ]

**Table 3 : Comparison of Test and Reference Tablets (molar ratio is 1:2.35 as rosuvastatin calcium : sodium carbonate anhydrous)**

| Time (Minutes) | Dissolved % | |
|---|---|---|
| | Crestor ® 40 mg Film Tablet (Reference) | Rosuvastatin Calcium 40 mg Film Tablet (Test) |
| 5 | 18,1 | 17,4 |
| 10 | 41,6 | 50,9 |
| 15 | 55,1 | 73,6 |
| 20 | 64,1 | 81,9 |
| 30 | 75,9 | 89,5 |
| 45 | 85,2 | 93,2 |
| 60 | 89,8 | 95,8 |

**Example 4**

Rosuvastatin calcium test tablet includes sodium carbonate anhydrous as within the molar ratio in the range of 1 : 4.71 (Rosuvastatin calcium : sodium carbonate anhydrous) is released in 0,1 N HCl environment under conditions of 900 ml of a dissolution medium at 37°C $\pm$0.5°C, USP method 1 (basket), 100 rpm basket speed wherein tablet exhibits a dissolution profile (Figure 4 and Table 4). At the same time sodium carbonate anhydrous is 5.16 % by weight of tablet. Under mentioned conditions f2 value is 22,1 .
[Table 4]
[Table ]

**Table 4 : Comparison of Test and Reference Tablets (molar ratio is 1:4.71 as rosuvastatin calcium : sodium carbonate anhydrous)**

| Minutes | Dissolved % | |
|---|---|---|
| | Crestor ® 40 mg Film Tablet (Reference) | Rosuvastatin Calcium 40 mg Film Tablet (Test) |
| 5 | 18,1 | 80,6 |
| 10 | 41,6 | 90,3 |
| 15 | 55,1 | 93,1 |
| 20 | 64,1 | 94,5 |
| 30 | 75,9 | 95,4 |
| 45 | 85,2 | 95,6 |
| 60 | 89,8 | 95,6 |

**Claims**

**1.** A pharmaceutical composition comprising

    i) rosuvastatin calcium:
    ii) an alkali metal carbonate or bicarbonate or mixtures thereof, and
    iii) optionally one or more pharmaceutically acceptable excipients

**characterized in that** rosuvastatin calcium and an alkali metal carbonate or bicarbonate or a mixture thereof are present in a molar ratio in the range of 1:0.43-1.75, wherein the composition provides a dissolution profile of rosuvastatin calcium in 0.1 N HCl in which rosuvastatin calcium is released in the range of 15 % to 25 % in 5 minutes, 40 % to 50 % in 10 minutes, 60 % to 70 % in 15 minutes, 70 % to 80 % in 20 minutes, 80 % to 90 % in 30 minutes, 85 % to 95 % in 45 minutes and 90 to 100 % in 60 minutes after association of 900 ml of a dissolution medium at 37°C $\pm$ 0.5°C and USP method 1 (basket) and basket speed is 100 rpm.

2. The composition of claim 1, wherein the alkali metal in the alkali metal carbonate or bicarbonate is selected from Na or K.

3. The composition of claim 2, wherein the alkali metal carbonate may be selected from $Na_2CO_3$ anhydrous or $K_2CO_3$ anhydrous or mixtures thereof.

4. The composition of claim 1, wherein the pharmaceutically acceptable excipient may be selected from a group consisting of filler, disintegrating agent and a lubricant.

5. The composition of claim 1, wherein the pharmaceutical composition is an oral dosage form.

6. The composition of claim 5, wherein the oral dosage form is a tablet

7. The composition of claim 1, wherein the pharmaceutical composition exhibits a dissolution profile as less than or equal to 85 % of the total amount of rosuvastatin calcium is released in 30 minutes.

8. The composition of claim 1, wherein the alkali metal carbonate orbicarbonate is present in the range of 0.5 % to 2 % by weight of pharmaceutical composition.


**Patentansprüche**

1. Eine pharmazeutische Zusammensetzung umfassend

    i) Rosuvastatin Calcium;
    ii) ein AlkaliMetall-Carbonat oder -bicarbonat oder Mischungen davon, und
    iii) Optional einer oder mehrere pharmazeutisch annehmbare Hilfsstoffe

**dadurch gekennzeichnet, dass** Rosuvastatin Calcium und ein AlkaliMetall-Carbonat oder - bicarbonat oder eine Mischung daraus in einem Molverhältnis im Bereich von 1 :0.43-1.75, bei dem die Zusammensetzung bietet Auflösung Profil der Rosuvastatin Calcium in 0.1 N HCl, wobei Rosuvastatin Calcium freigesetzt 15 % bis 25 % in 5 Minuten, 40 % bis 50 % in 10 Minuten, 60 bis 70 % in 15 Minuten, 70 % bis 80 % in 20 Minuten, 80 % bis 90 % in 30 Minuten, 85 % bis 95 % in 45 Minuten und 90 bis 100 % in 60 Minuten.

2. Die Zusammensetzung gemäß Anspruch 1, wobei das AlkaliMetall Carbonat oder Bicarbonat aus Na oder K.

3. Die Zusammensetzung gemäß Anspruch 2, wobei das AlkaliMetall Carbonat kann sein ausgewählt aus $Na_2CO_3$ wasserfreien oder $K_2CO_3$ wasserfreien oder Mischungen daraus.

4. Die Zusammensetzung gemäß Anspruch 1, wobei der pharmazeutisch zulässige Arzneistoffträger kann von einer Gruppe bestehend aus Füllstoffen, Sprengmitteln und Gleitmitteln ausgewählt werden.

5. Die Zusammensetzung gemäß Anspruch 1, die pharmazeutische Zusammensetzung ist eine orale Darreichungsform.

6. Die Zusammensetzung gemäß Anspruch 5, orale Darreichungsform ist eine Tablette.

7. Die Zusammensetzung gemäß Anspruch 1, wobei die pharmazeutische Zusammensetzung zeigt ein Auflösung Profil weniger als oder gleich 85% der Gesamtmenge der Rosuvastatin calcium wird in 30 Minuten freigesetzt.

8. Die Zusammensetzung gemäß Anspruch 1, AlkaliMetall Carbonat oder -bicarbonat ist im Bereich von 0.5 % bis 2 % des Gewichts der pharmazeutischen Zusammensetzung.

## Revendications

1.  Un composition pharmaceutique comprenant

    i) rosuvastatine calcique;
    ii) carbonate ou bicarbonate d'un métal alcalin ou leurs mélanges et
    iii) éventuellement un ou plusieurs excipients pharmaceutiquement acceptable charactérisé en ce que rosuvastatine calcique et carbonate ou bicarbonate d'un métal alcalin ou leurs mélanges sont présents dans un rapport molaire dans la gamme de 1:0.43-1.75, dans laquelle la composition fournit un profil de dissolution de rosuvastatine calcique dans HCl 0.1 N dans la quelle rosuvastatine calcique est libérée dans la gamma de de 15% à 25 % en 5 minutes, 40 % à 50 % en 10 minutes, 60 % à 70 % en 15 minutes, 70 % à 80 % en 20 minutes, 80% à 90% en 30 minutes, 85% à 95% en 45 minutes, 90 % à 100 % en 60 minutes.

2.  La composition de la revendication 1, dans lequel le carbonate de métal alcalin ou de bicarbonate est choisi parmi Na ou K.

3.  La composition de la revendication 2, dans lequel le carbonate de métal alcalin peut être choisi parmi $Na_2CO_3$ ou $K_2CO_3$ anhydre anhydres ou des mélanges de ceux-ci.

4.  La composition de la revendication 1, dans laquelle l'excipient pharmaceutiquement acceptable peut être choisi dans un groupe qui contient un agent diluant, un agent désintégrant et un lubrifiant.

5.  La composition de la revendication 1, dans laquelle la composition pharmaceutique est une forme galénique orale.

6.  La composition de la revendication 5, dans lequel la forme galénique orale est un comprimé.

7.  La composition de la revendication 1, dans laquelle la composition pharmaceutique présente un profil de dissolution où une quantité inférieure ou égale au 85% de la quantité totale de rosuvastatine calcique est libéré en 30 minutes.

8.  La composition de la revendication 1, dans lequel le carbonate ou le bicarbonate du métal alcalin est présent dans la gamme de 0,5% à 2% en poids de la composition pharmaceutique.

[Fig. 1]

Rosuvastatin Calcium 40 mg Film Tablet (Test) and Crestor ® 40 mg Film Tablet (Reference)

[Fig. 2]

Rosuvastatin Calcium 40 mg Film Tablet (Test) and Crestor ® 40 mg Film Tablet (Reference)

[Fig. 3]

Rosuvastatin Calcium 40 mg Film Tablet (Test) and Crestor ® 40 mg Film Tablet (Reference)

[Fig. 4]

Rosuvastatin Calcium 40 mg Film Tablet (Test) and Crestor ® 40 mg Film Tablet (Reference)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 521471 A
- EP 0547000 A
- EP 1223918 A
- EP 1905424 A
- WO 2008062476 A